(19) Europäisches Patentamt
European Patent Office
Office européen des brevets

(11) Veröffentlichungsnummer: **0 047 420**
**A2**

(12) **EUROPÄISCHE PATENTANMELDUNG**

(21) Anmeldenummer: **81106547.3**

(22) Anmeldetag: **24.08.81**

(51) Int. Cl.[3]: **C 07 D 249/08**, C 07 D 233/60, A 01 N 43/64, A 01 N 43/50 // C07C49/255

(30) Priorität: **06.09.80 DE 3033592**

(43) Veröffentlichungstag der Anmeldung: **17.03.82 Patentblatt 82/11**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Krämer, Wolfgang, Dr., Am Eckbusch 39/45, D-5600 Wuppertal 1 (DE)**
Erfinder: **Büchel, Karl Heinz, Prof. Dr., Dabringhausenerstrasse 42, D-5093 Burscheid (DE)**
Erfinder: **Stetter, Jörg, Dr., Gellertweg 4, D-5600 Wuppertal 1 (DE)**
Erfinder: **Frohberger, Paul-Ernst, Dr., Willi-Baumeister-Strasse 5, D-5090 Leverkusen (DE)**
Erfinder: **Brandes, Wilhelm, Dr., Eichendorffstrasse 3, D-5653 Leichlingen (DE)**

(54) **Fluorierte 1-Azolyl-butan-Derivate, Verfahren zu ihrer Herstellung und ihre Verwendung als Fungizide.**

(57) Die Erfindung betrifft neue fluorierte 1-Azolyl-butan-Derivate, ein Verfahren zu ihrer Herstellung und ihre Verwendung als Fungizide.

Die Verbindungen der allgemeinen Formel

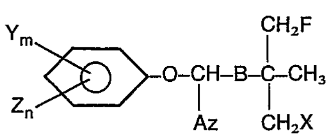

(I)

in welcher

Az, B, X, Y, Z, m und n die in der Beschreibung angegebene Bedeutung besitzen, werden erhalten, wenn man Halogenetherketone mit Imidazol oder 1,2,4-Triazol in Gegenwart eines Säurebindemittels und gegebenenfalls in Gegenwart eines Verdünnungsmittels umsetzt und gegebenenfalls die erhaltenen Keto-Derivate nach bekannten Methoden in üblicher Weise reduziert.

Die Wirkstoffe sind für den Gebrauch als Pflanzenschutzmittel geeignet und können mit besonders gutem Erfolg zur Bekämpfung von Getreidekrankheiten sowie zur Bekämpfung des Gurkenmehltaus, des Apfelmehltaus und des Apfelschorfs eingesetzt werden.

EP 0 047 420 A2

0047420

BAYER AKTIENGESELLSCHAFT 5090 Leverkusen, Bayerwerk
Zentralbereich Slr/W
Patente, Marken und Lizenzen Ia

05.09.80

Fluorierte 1-Azolyl-butan-Derivate, Verfahren zu ihrer Herstellung und ihre Verwendung als Fungizide

Die vorliegende Erfindung betrifft neue fluorierte 1-Azolyl-butan-Derivate, ein Verfahren zu ihrer Herstellung und ihre Verwendung als Fungizide.

Es ist bereits bekannt geworden, daß chlorierte und bromierte 1-Imidazolyl- und Triazolyl-butan-Derivate gute fungizide Eigenschaften aufweisen (vergleiche DE-OS 26 32 602 [LeA 17 274] und DE-OS 26 32 603 [LeA 17 273] ). Die Wirkung dieser Verbindungen ist jedoch, insbesondere bei niedrigen Aufwandmengen und -konzentrationen, nicht immer ganz befriedigend.

Es wurden neue fluorierte 1-Azolyl-butan-Derivate der allgemeinen Formel

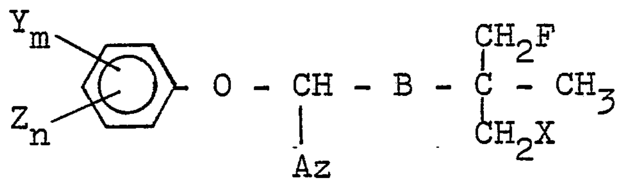

(I)

in welcher

Az für Imidazol-1-yl, 1,2,4-Triazol-1-yl oder 1,2,4-Triazol-4-yl steht,

B für die Ketogruppe oder die CH(OH)-Gruppierung steht,

X für Wasserstoff oder Fluor steht,

Y für Alkoxy, Alkylthio, Halogenalkyl, Halogenalkoxy oder Halogenalkylthio steht,

Z für Halogen oder Alkyl steht,

m für die Zahlen 1, 2 oder 3 steht und

n für die Zahlen 0, 1 oder 2 steht,

sowie deren physiologisch verträglichen Säureadditions-Salze und Metallsalz-Komplexe gefunden.

Diejenigen Verbindungen der Formel (I), in welchen B für die CH(OH)-Gruppierung steht, besitzen zwei asymmetrische Kohlenstoffatome; sie können deshalb in zwei geometrisch Isomeren (threo- und erythro-Form) vorliegen, die in unterschiedlichen Mengenverhältnissen anfallen können. In beiden Fällen liegen sie als optische Isomeren vor.

Sämtliche Isomeren werden erfindungsgemäß beansprucht.

Weiterhin wurde gefunden, daß man die fluorierten 1-Azolyl-butan-Derivate der Formel (I) erhält, wenn man Halogenetherketone der Formel

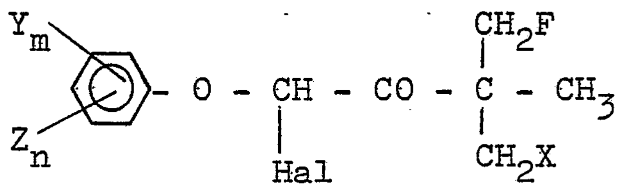
(II)

in welcher

X,Y,Z,n und m die oben angegebene Bedeutung haben und

Hal für Halogen, vorzugsweise Chlor oder Brom, steht,

mit Imidazol oder 1,2,4-Triazol in Gegenwart eines Säurebindemittels und gegebenenfalls in Gegenwart eines Verdünnungsmittels umsetzt und gegebenenfalls die erhaltenen Keto-Derivate nach bekannten Methoden in üblicher Weise reduziert.

An die so erhaltenen Verbindungen der Formel (I) können gegebenenfalls anschließend eine Säure oder ein Metallsalz addiert werden.

Die neuen fluorierten 1-Azolyl-butan-Derivate weisen starke fungizide Eigenschaften auf. Dabei zeigen überraschenderweise die erfindungsgemäßen Verbindungen eine höhere Wirkung als die aus dem Stand der Technik bekannten chlorierten und bromierten 1-Imidazolyl- und Triazolylbutan-Derivate, die chemisch und wirkungsmäßig naheliegendste Verbindungen sind. Die erfindungsgemäßen Stoffe stellen somit eine Bereicherung der Technik dar.

Die erfindungsgemäßen fluorierten 1-Azolyl-butan-Derivate sind durch die Formel (I) allgemein definiert. In dieser Formel steht Y vorzugsweise für Alkoxy und Alkylthio mit jeweils 1 bis 4 Kohlenstoffatomen sowie für Halogenalkyl, Halogenalkoxy und Halogenalkylthio mit jeweils 1 bis 2 Kohlenstoff- und 1 bis 5 gleichen oder verschiedenen Halogenatomen, wie insbesonderen Fluor- und Chloratomen. Z steht vorzugsweise für Halogen und Alkyl mit 1 bis 4 Kohlenstoffatomen. Az, B, X und die Indizes m und n haben vorzugsweise die in der Erfindungsdefinition angegebene Bedeutung.

Besonders bevorzugt sind diejenigen fluorierten 1-Azolylbutan-Derivate, in denen Y für Methoxy, Ethoxy, Isopropoxy, tert.-Butyloxy, Methylthio, Trifluormethyl, Chlordifluormethyl, Dichlorfluormethyl, Trichlormethyl, Trifluormethoxy oder Trifluormethylthio steht; Z für Fluor, Chlor oder Methyl steht; und Az, B, X sowie die Indizes m und n die in der Erfindungsdefinition angegebene Bedeutung haben.

Im einzelnen seien außer den bei den Herstellungsbeispielen genannten Verbindungen die folgenden Verbindungen der allgemeinen Formel (I) genannt:

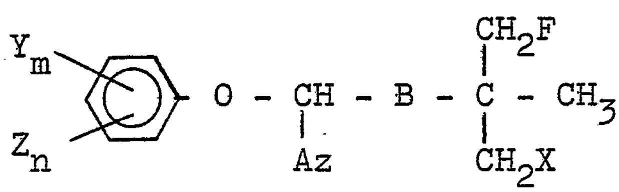

(I)

| $Y_m$ | $Z_n$ | B | X | Az |
|---|---|---|---|---|
| 3,5-$(CF_3)_2$ | - | CO | F | 1,2,4-Triazol-1-yl |
| 4-$SCF_3$ | - | CO | F | 1,2,4-Triazol-1-yl |
| 4-$OCF_3$ | - | CO | F | 1,2,4-Triazol-1-yl |
| 4-$SCH_3$ | - | CO | F | 1,2,4-Triazol-1-yl |
| 4-$SCF_3$ | 3-$CH_3$ | CO | F | 1,2,4-Triazol-1-yl |
| 4-$CF_3$ | 2-Cl | CO | F | 1,2,4-Triazol-1-yl |
| 4-$SCF_3$ | 2,6-$Cl_2$ | CO | F | 1,2,4-Triazol-1-yl |
| 3,5-$(CF_3)_2$ | - | CH(OH) | F | 1,2,4-Triazol-1-yl |
| 4-$SCF_3$ | - | CH(OH) | F | 1,2,4-Triazol-1-yl |
| 4-$OCF_3$ | - | CH(OH) | F | 1,2,4-Triazol-1-yl |
| 4-$SCH_3$ | - | CH(OH) | F | 1,2,4-Triazol-1-yl |
| 4-$SCF_3$ | 3-$CH_3$ | CH(OH) | F | 1,2,4-Triazol-1-yl |
| 4-$CF_3$ | 2-Cl | CH(OH) | F | 1,2,4-Triazol-1-yl |
| 4-$SCF_3$ | 2,6-$Cl_2$ | CH(OH) | F | 1,2,4-Triazol-1-yl |
| 3,5-$CF_3$ | - | CO | H | 1,2,4-Triazol-1-yl |
| 4-$SCF_3$ | - | CO | H | 1,2,4-Triazol-1-yl |
| 4-$OCF_3$ | - | CO | H | 1,2,4-Triazol-1-yl |
| 4-$SCH_3$ | - | CO | H | 1,2,4-Triazol-1-yl |
| 4-$SCF_3$ | 3-$CH_3$ | CO | H | 1,2,4-Triazol-1-yl |
| 4-$CF_3$ | 2-Cl | CO | H | 1,2,4-Triazol-1-yl |
| 4-$SCF_3$ | 2,6-$Cl_2$ | CO | H | 1,2,4-Triazol-1-yl |
| 3,5-$(CF_3)_2$ | - | CH(OH) | H | 1,2,4-Triazol-1-yl |
| 4-$SCF_3$ | - | CH(OH) | H | 1,2,4-Triazol-1-yl |
| 4-$OCF_3$ | - | CH(OH) | H | 1,2,4-Triazol-1-yl |
| 4-$SCH_3$ | - | CH(OH) | H | 1,2,4-Triazol-1-yl |
| 4-$SCF_3$ | 3-$CH_3$ | CH(OH) | H | 1,2,4-Triazol-1-yl |
| 4-$CF_3$ | 2-Cl | CH(OH) | H | 1,2,4-Triazol-1-yl |
| 4-$SCF_3$ | 2,6-$Cl_2$ | CH(OH) | H | 1,2,4-Triazol-1-yl |

| $Y_m$ | $Z_n$ | B | X | Az |
|---|---|---|---|---|
| 3,5-$(CF_3)_2$ | - | CO | F | Imidazol-1-yl |
| 4-$SCF_3$ | - | CO | F | Imidazol-1-yl |
| 4-$OCF_3$ | - | CO | F | Imidazol-1-yl |
| 4-$SCH_3$ | - | CO | F | Imidazol-1-yl |
| 4-$SCF_3$ | 3-$CH_3$ | CO | F | Imidazol-1-yl |
| 4-$CF_3$ | 2-Cl | CO | F | Imidazol-1-yl |
| 4-$SCF_3$ | 2,6-$Cl_2$ | CO | F | Imidazol-1-yl |
| 3,5-$(CF_3)_2$ | - | CH(OH) | F | Imidazol-1-yl |
| 4-$SCF_3$ | - | CH(OH) | F | Imidazol-1-yl |
| 4-$OCF_3$ | - | CH(OH) | F | Imidazol-1-yl |
| 4-$SCH_3$ | - | CH(OH) | F | Imidazol-1-yl |
| 4-$SCF_3$ | 3-$CH_3$ | CH(OH) | F | Imidazol-1-yl |
| 4-$CF_3$ | 2-Cl | CH(OH) | F | Imidazol-1-yl |
| 4-$SCF_3$ | 2,6-$Cl_2$ | CH(OH) | F | Imidazol-1-yl |
| 3,5-$CF_3$ | - | CO | H | Imidazol-1-yl |
| 4-$SCF_3$ | - | CO | H | Imidazol-1-yl |
| 4-$OCF_3$ | - | CO | H | Imidazol-1-yl |
| 4-$SCH_3$ | - | CO | H | Imidazol-1-yl |
| 4-$SCF_3$ | 3-$CH_3$ | CO | H | Imidazol-1-yl |
| 4-$CF_3$ | 2-Cl | CO | H | Imidazol-1-yl |
| 4-$SCF_3$ | 2,6-$Cl_2$ | CO | H | Imidazol-1-yl |
| 3,5-$(CF_3)_2$ | - | CH(OH) | H | Imidazol-1-yl |
| 4-$SCF_3$ | - | CH(OH) | H | Imidazol-1-yl |
| 4-$OCF_3$ | - | CH(OH) | H | Imidazol-1-yl |
| 4-$SCH_3$ | - | CH(OH) | H | Imidazol-1-yl |
| 4-$SCF_3$ | 3-$CH_3$ | CH(OH) | H | Imidauol-1-yl |
| 4-$CF_3$ | 2-Cl | CH(OH) | H | Imidazol-1-yl |
| 4-$SCF_3$ | 2,6-$Cl_2$ | CH(CH) | H | Imidazol-1-yl |

| $Y_n$ | $Z_n$ | B | X | Az |
|---|---|---|---|---|
| $3-CF_3$ | - | CO | H | 1,2,4-Triazol-1-yl |
| $4-OCH_3$ | - | CO | H | 1,2,4-Triazol-1-yl |
| $3-CF_3$ | - | CH(OH) | H | 1,2,4-Triazol-1-yl |
| $4-OCH_3$ | - | CH(OH) | H | 1,2,4-Triazol-1-yl |
| $3-CF_3$ | - | CO | H | Imidazol-1-yl |
| $4-OCH_3$ | - | CO | H | Imidazol-1-yl |
| $3-CF_3$ | - | CH(OH) | H | Imidazol-1-yl |
| $4-OCH_3$ | - | CH(OH) | H | Imidazol-1-yl |

Verwendet man beispielsweise 1-Brom-1-(3-trifluormethyl-phenoxy)-3,3-dimethyl-4-fluor-butan-2-on und 1,2,4-Triazol als Ausgangsstoffe, so kann der Reaktionsablauf durch das folgende Formelschema wiedergegeben werden:

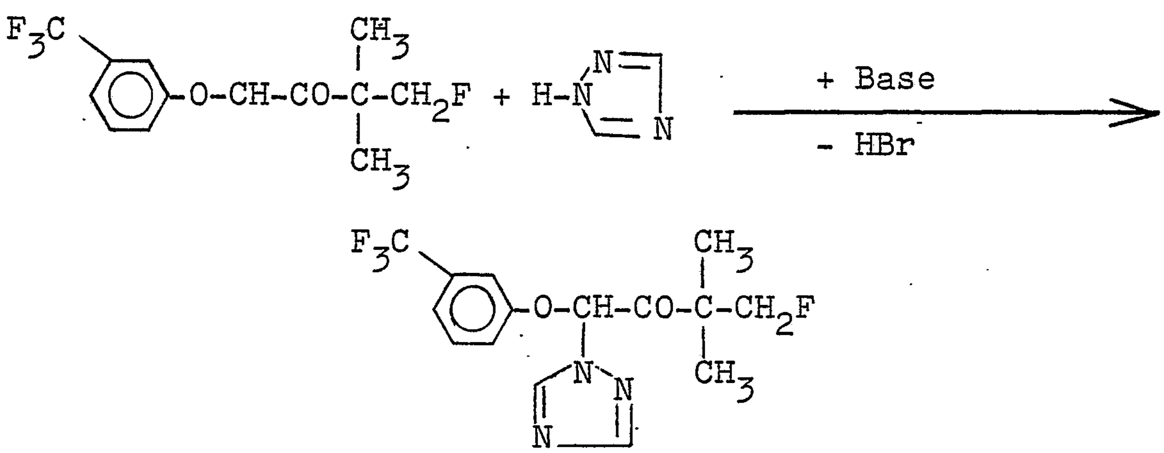

Verwendet man beispielsweise 3,3-Dimethyl-4-fluor-1-(1,2,4-triazol-1-yl)-1-(3-trifluormethylphenoxy)-butan-2-on und Natriumborhydrid als Ausgangsstoffe, so kann der Reaktionsablauf der Reduktion durch das folgende Formelschema wiedergegeben werden:

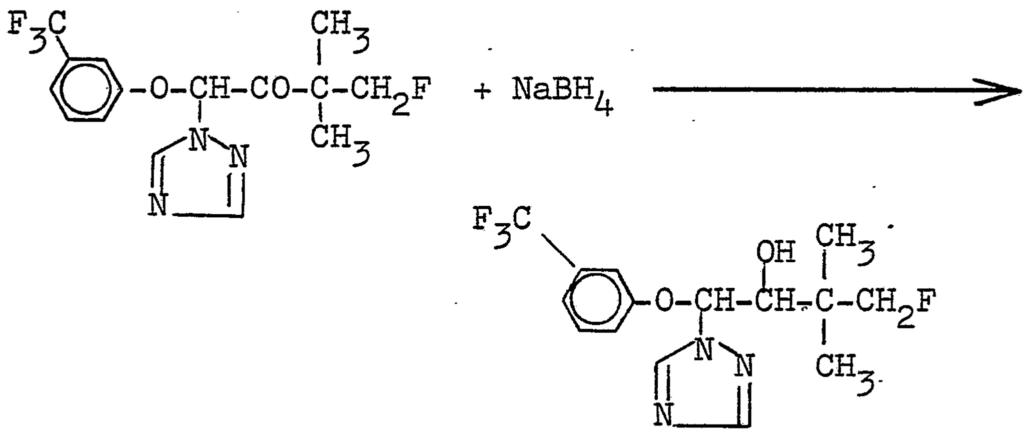

Die bei der Durchführung des erfindungsgemäßen Verfahrens als Ausgangsstoffe zu verwendenden Halogenetherketone sind durch die Formel (II) allgemein definiert. In dieser Formel stehen X,Y,Z und die Indizes m und n vorzugsweise für diejenigen Reste, die bereits im Zusammenhang mit der Beschreibung der erfindungsgemäßen Stoffe der Formel (I) vorzugsweise für diese Substituenten genannt wurden.

Die Halogenetherketone der Formel (II) sind noch nicht bekannt. Sie können jedoch nach bekannten Verfahren (vergleiche z.B. DE-OS 26 32 603 [LeA 17 273] ) erhalten werden, indem man z.B. bekannte Phenole der Formel

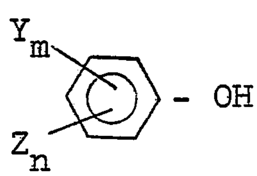

(III)

in welcher

Y,Z,m und n die oben angegebene Bedeutung haben,

mit einem bekannten (vergleiche DE-OS 28 43 767 [LeA 18 985] ) Halogenketon der Formel

$$Hal - CH_2 - CO - \underset{\displaystyle CH_2X}{\overset{\displaystyle CH_2F}{C}} - CH_3 \qquad (IV)$$

in welcher

Hal und X die oben angegebene Bedeutung haben,

umsetzt. Das noch verbliebene aktive Wasserstoffatom wird anschließend in üblicher Weise gegen Halogen ausgetauscht (vergleiche auch die Herstellungsbeispiele).

Für die erfindungsgemäße Umsetzung kommen als Verdünnungsmittel inerte organische Lösungsmittel infrage. Hierzu gehören vorzugsweise Ketone, wie Diethylketon und insbesondere Aceton und Methylethylketon; Nitrile, wie Propionitril, insbesondere Acetonitril; Alkohole, wie Ethanol oder Isopropanol; Ether, wie Tetrahydrofuran oder Dioxan; Benzol; Toluol; Formamide; wie insbesondere Dimethylformamid; und halogenierte Kohlenwasserstoffe.

Die erfindungsgemäße Umsetzung wird in Gegenwart eines Säurebinders vorgenommen. Man kann alle üblicherweise verwendbaren anorganischen oder organischen Säurebinder zugeben, wie Alkalicarbonate, beispielsweise Natriumcarbonat und Natriumhydrogencarbonat, oder wie niedere tertiäre Alkylamine, Cycloalkylamine oder Aralkylamine, beispielsweise Triethylamin, N,N-Dimethylcyclohexylamin, Diyclohexylamin, N,N-Dimethylbenzylamin, weiterhin Pyridin und Diazabicyclooctan.

Vorzugsweise verwendet man einen entsprechenden Ueberschuß an Triazol bzw. Imidazol.

Die Reaktionstemperaturwn können in einem größeren Bereich variiert werden. Im allgemeinen arbeitet man zwischen etwa 20 bis etwa 150°C, vorzugsweise bei 60 bis 120°C. Bei Anwesenheit eines Lösungsmittels wird zweckmäßigerweise beim Siedepunkt des jeweiligen Lösungsmittels gearbeitet.

Bei der Durchführung des erfindungsgemäßen Verfahrens setzt man auf 1 Mol der Verbindungen der Formel (II) vorzugsweise 2 Mol Azol und 1 bis 2 Mol Säurebinder ein. Zur Isolierung der Verbindungen der Formel (I ) wird das Lösungsmittel abdestilliert, der Rückstand mit einem organischen Solvens aufgenommen und mit Wasser gewaschen. Die organische Phase wird über Natriumsulfat getrocknet und im Vakuum vom Lösungsmittel befreit. Der Rückstand wird durch Destillation bzw. Umkristallisation oder Salzbildung und Umkristallisation gereinigt.

Die erfindungsgemäße Reduktion erfolgt in üblicher Weise, wie z.B. durch Umsetzung mit komplexen Hydriden, gegebenenfalls in Gegenwart eines Verdünnungsmittels, oder durch Umsetzung mit Aluminium-isopropylat in Gegenwart eines Verdünnungsmittels.

Arbeitet man mit komplexen Hydriden, so kommen als Verdünnungsmittel für die erfindungsgemäße Umsetzung polare organische Lösungsmittel infrage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei 0 bis 30°C, vorzugsweise bei 0 bis 20°C durchgeführt. Hierzu setzt man auf 1 Mol des Ketons der Formel (I) etwa 1 Mol eines komplexen Hydrids, wie Natriumborhydrid oder Lithiumalanat, ein. Zur Isolierung der reduzierten Verbindungen der Formel (I) wird der Rückstand in verdünnter Salzsäure aufgenommen, anschliessend alkalisch gestellt und mit einem organischen Lösungsmittel extrahiert. Die weitere Aufarbeitung erfolgt in üblicher Weise.

Arbeitet man mit Aluminium-isopropylat, so kommen als Verdünnungsmittel für die erfindungsgemäße Umsetzung bevorzugt Alkohole, wie Isopropanol, oder inerte Kohlenwasserstoffe, wie Toluol, infrage. Die Reaktionstemperaturen können wiederum in einem größeren Bereich variiert werden; im allgemeinen arbeitet man zwischen 20 und 120°C, vorzugsweise bei 50 bis 100°C. Zur Durchführung der Reaktion setzt man auf 1 Mol des Ketons der Formel (I) etwa 0,3 bis 2 Mol Aluminium-isopropylat ein. Zur Isolierung der reduzierten Verbindungen der Formel (I) wird das überschüssige Lösungsmittel im Vakuum entfernt und die entstandenen Aluminium-Verbindungen mit verdünnter Schwefelsäure oder Natronlauge zersetzt. Die weitere Aufarbeitung erfolgt in üblicher Weise.

Zur Herstellung der Säureadditionssalze der Verbindungen der Formel (I) kommen alle physiologisch verträglichen Säuren infrage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure , Maleinsäure, Bernsteinsäure, Fumarsäure, Weinsäure, Zitronensäure, Salizylsäure. Sorbinsäure, Milchsäure, sowie Sulfonsäuren, wie z.B. p-Toluolsulfonsäure und 1,5-Naphthalindisulfonsäure.

Die Salze der Verbindungen der Formel (I) können in einfacher Weise nach üblichen Salzbildungsmethoden, z.B. durch Lösen einer Verbindung der Formel (I) in einem geeigneten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel oder durch Umkristallisation gereinigt werden.

Zur Herstellung von Metallsalz-Komplexen der Verbindungen der Formel (I) kommen vorzugsweise Salze von Metallen der II. bis IV.-Haupt- und der I. und II. sowie IV. bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich von physiologischen Säuren ableiten. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure.

Die Metallkomplexe der Verbindungen der Formel (I) können in einfacher Weise nach üblichen Verfahren erhalten werden, so z.B. durch Lösen des Metallsalzes in Alkohol, z.B. Ethanol, und Hinzufügen zur Verbindung der Formel (I). Man kann Metallsalzkomplexe in bekannter Weise, z.B. durch Abfiltrieren, isolieren und gegebenenfalls durch Umkristallisation reinigen.

Die erfindungsgemäßen Wirkstoffe weisen starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie Getreidemehltau und Gerstenmehltau, sowie zur Bekämpfung des Gurkenmehltaus (Erysiphe cichoracearum) des Apfelmehltaus (Podosphaera leucotricha) und des Apfelschorfs (Fusicladium dendriticum) eingesetzt werden. Hervorzuheben ist, daß die erfindungsgemäßen Stoffe nicht nur eine protektive Wirkung entfalten, sondern auch systemisch wirksam sind. So gelingt es, Pflanzen gegen Pilzbefall zu schützen, wenn man die Wirkstoffe über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

0047420

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Herstellungsbeispiele

Beispiel 1

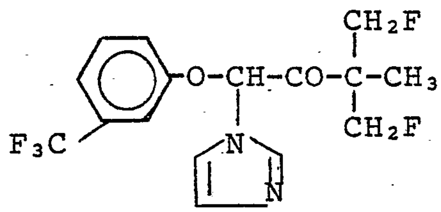

135g (0,36 Mol) 3,3-Bisfluormethyl-1-brom-1-(3-trifluormethyl-phenoxy)-butan-2-on in 400 ml Aceton werden zu 24,5g (0,36 Mol) Imidazol und 55,3g (0,4 Mol) Kaliumcarbonat in 400ml Aceton getropft. Man läßt 6 Stunden bei 50°C rühren und engt dann durch Abdestillieren des Lösungsmittels im Wasserstrahlvakuum ein. Der oelige Rückstand wird in 800 ml Methylenchlorid aufgenommen und zweimal mit je 2000ml Wasser gewaschen, die organische Phase mit Aktivkohle ausgerührt, abfiltriert, über Natriumsulfat getrocknet und das Lösungsmittel abdestilliert. Der ölige Rückstand wird im Hochvakuum destilliert. Man erhält 63,4g (48,8 % der Theorie) 3,3-Bisfluormethyl-1-(imidazol-1-yl)-1-(3-trifluormethyl-phenoxy)-butan-2-on vom Siedepunkt 169-73°C/ 0,1 Torr.

Herstellung des Vorproduktes

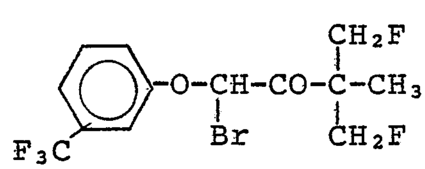

210g (0,71 Mol) 3,3-Bisfluormethyl-1-(3-trifluormethyl-phenoxy)-butan-2-on werden in 500ml Methylenchlorid gelöst und bei 20 bis 30°C unter Rühren tropfenweise

mit 36,1 ml (0,71 Mol)Brom in 40 ml Methylenchlorid versetzt. Es wird 2 Stunden bei 20°C nachgerührt und durch Abdestillieren des Lösungsmittels im Vakuum eingeengt. Der ölige Rückstand wird im Hochvakuum destilliert. Man erhält 235g 3,3-Bisfluormethyl-1-brom-1-(3-trifluormethylphenoxy)-butan-2-on vom Siedepunkt 110-16°C/0,06 Torr.

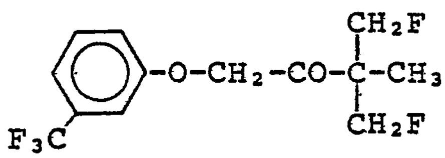

Zu einer gerührten Mischung von 162g (1 Mol) 3-Trifluormethylphenol und 180g (1,3 Mol) gepulvertem Kaliumcarbonat in 12ooml Aceton werden bei 20 bis 30°C 215g (1 Mol) 3,3-Bisfluormethyl-1-brom-butan-2-on zugetropft. Man läßt 6 Stunden bei 50°C rühren, das anorganische Salz wird abfiltriert und das Filtrat eingeengt. Der Rückstand wird im Hochvakuum destilliert. Man erhält 210g 3,3-Bisfluormethyl-1-(3-trifluormethylphenoxy)-butan-2-on vom Siedepunkt 114-18°C/0,6 Torr.

<u>Beispiel 2</u>

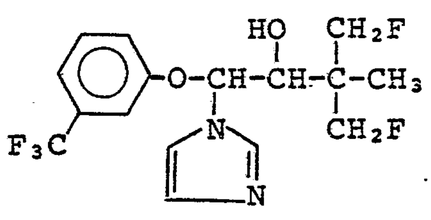

63,7g (0,176 Mol) 3,3-Bisfluormethyl-1-(imidazol-1-yl)-1-(3-trifluormethylphenoxy)-butan-2-on (Beispiel 1) werden in 250 ml Methanol gelöst und portionsweise mit

3g (0,08 Mol) Natriumborhydrid versetzt. Man läßt die Reaktionslösung 1 Stunde nachrühren und stellt sie dann mit konzentrierter Salzsäure auf einen pH-Wert von 3 ein. Nach Abdestillieren des Lösungsmittels im Vakuum wird der Rückstand mit Wasser versetzt und mit Methylenchlorid ausgeschüttelt. Die vereinigten organischen Phasen werden über Natriumsulfat getrocknet und im Vakuum eingeengt. Man erhält 44,5g (70 % der Theorie) 3,3-Bisfluormethyl-1-(imidazol-1-yl)-1-(3-trifluormethylphenoxy)-butan-2-ol vom Schmelzpznkt 107-20°C.

Beispiel 3

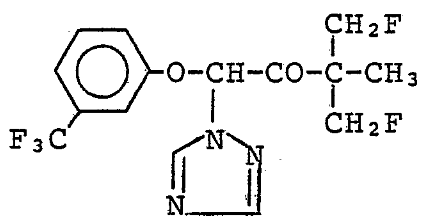

100g (0,267 Mol) 3,3-Bisfluormethyl-1-brom-1-(3-trifluormethylphenoxy)-butan -2-on und 37,2g (0.54 Mol) 1,2,4-Triazol werden in 600 ml Acetonitril 6 Stunden unter Rühren auf 65°C erhitzt. Danach wird das Lösungsmittel im Wasserstrahlvakuum abdestilliert, der Rückstand in 800ml Methylenchlorid aufgenommen und zweimal mit je 1000 ml Wasser gewaschen. Die organische Phase wird über Natriumsulfat getrocknet und eingeengt. Der ölige Rückstand wird im Hochvakuum destilliert. Man erhält 80g (82,4 % der Theorie) 3,3-Bisfluormethyl-1-(1,2,4-triazol-1-yl)-1-(3-trifluormethylphenoxy)-butan-2-on vom Siedepunkt 136-41°C/0,1 Torr.

0047420

In entsprechender Weise werden die nachfolgenden Beispiele der allgemeinen Formel

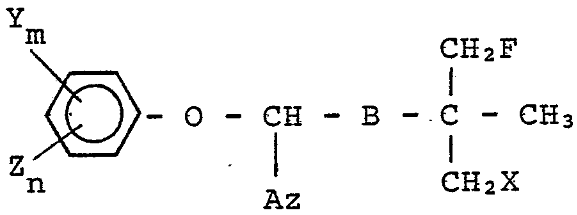

(I)

erhalten:

| Bsp. Nr. | $Y_m$ | $Z_n$ | B | X | Az | Schmelzp. (°C) bzw. Siedep. (°C (/Torr |
|---|---|---|---|---|---|---|
| 4 | 4-$OCH_3$ | - | CO | F | 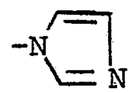 | 247-49 (x1/2 NDS) |
| 5 | 4-$OCH_3$ | - | CH(OH) | F | 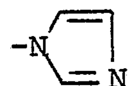 | 110-15 |
| 6 | 4-$OCH_3$ | - | CO | F | 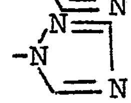 | Oel |
| 7 | 3-$CF_3$ | - | CH(OH) | F | 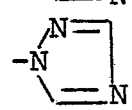 | 143-47 (A-Form) |
| 8 | 4-$OCH_3$ | - | CH(OH) | F | 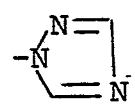 | 109-11 (A-Form) |

NDS = 1,5-Naphthalindisulfonsäure

A-Form= Eines der beiden möglichen geometrischen Isomeren

Verwendungsbeispiele

In den nachfolgenden Beispielen werden die nachstehend angegebenen Verbindungen als Vergleichssubstanzen eingesetzt:

(A) = 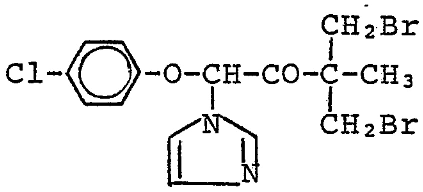 x 1/2 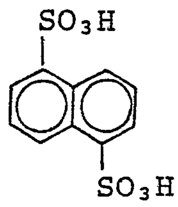

(B) = 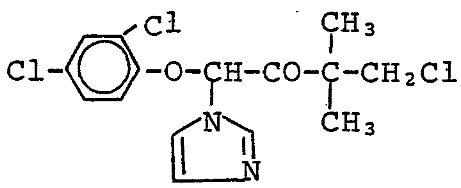

(C) = 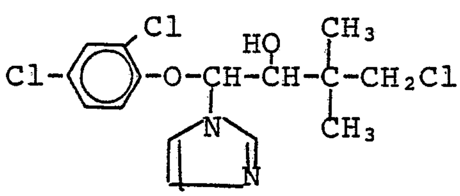

(D) = 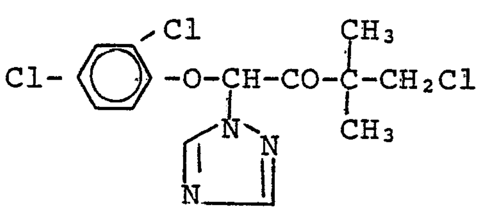

(E) = 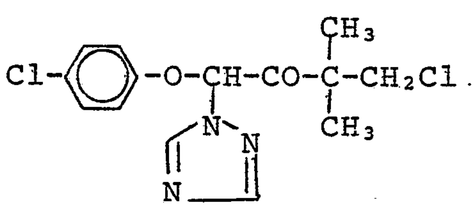

Beispiel A

Erysiphe-Test (Gerste) / protektiv /

Lösungsmittel= 100 Gewichtsteile Dimethylformamid

Emulgator = 0,25 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Ueberlegenheit in der Wirksamkeit gegenüber dem Stand der Technik zeigen bei diesem Test z.B. die Verbindungen gemäß folgender Herstellungsbeispiele= 1, 2, 3, 7, 8 und6.

Beispiel B

Gerstenmehltau-Test (Erysiphe graminis var.hordei)/ systemisch (pilzliche Getreidesproßkrankheit)

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 x 12 Korn in Blumentöpfe 2 cm tief in ein Gemisch aus einem Volumenteil Fruhstorfer Einheitserde und einem Volumenteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var.hordei bestäubt und bei 21-22°C und 80-90% rel.Luftfeuchte und 16-stündiger Belichtung weiter kultiviert. Innerhalb von 6 Tagen bilden sich an den Blättern die typischen Mehltaupusteln aus.
Der Befallsgrad wird in Prozent des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Eine deutliche Ueberlegenheit in der Wirksamkeit gegenüber dem Stand der Technik zeigen bei diesem Test z.B. die Verbindungen gemäß folgender Herstellungsbeispiele 1, 5, 3, 7, 8 und 6.

Beispiel C

Erysiphe-Test (Gurken)/ Protektiv

Lösungsmittel: 4,7 Gewichtsteile Aceton

Emulgator: 0,3 Gewichtsteile Alkyl-aryl-polyglykolether

Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält,
Mit der Spritzflüssigkeit bespritzt man junge Gurkenpflanzen mit etwa drei Laubblättern bis zur Tropfnässe. Die Gurkenpflanzen verbleiben zur Trocknung 24 Stunden im Gewächshaus. Dann werden sie zur Inokulation mit Konidien des Pilzes Erysiphe cichoracearum bestäubt. Die Pflanzen werden anschließend bei 23 bis 24°C und bei einer relativen Luftfeuchtigkeit von ca 75 % im Gewächshaus aufgestellt.
Nach 12 Tagen wird der Befall der Gurkenpflanzen bestimmt. Die erhaltenen Boniturwerte werden in Prozent Befall umgerechnet. 0 % bedeutet keinen Befall, 100 % bedeutet, daß die Pflanzen vollständig befallen sind.

Bei diesem Test zeigen z.B. die folgenden Verbindungen eine sehr gute Wirkung, die derjenigen der aus dem Stand der Technik bekannte Verbindung (A) überlegen ist:
Verbindungen gemäß Herstellungsbeispielen: 1.

Beispiel D

Podosphaera-Test (Apfel) / protektiv

Lösungsmittel: 4,7 Gewichtsteile Aceton

Emulgator : 0,3 Gewichtsteile Alkylarylpolyglykolether

Wasser : 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70 % im Gewächshaus. Anschließend werden sie durch Bestäuben mit Konidien des Apfelmehltauerregers (Podosphaera leucotricha) inokuliert und in ein Gewächshaus mit einer Temperatur von 21 bis 23 °C und einer relativen Luftfeuchtigkeit von 70 % gebraucht.

10 Tage nach der Inokulation wird der Befall der Sämlinge bestimmt. Die erhaltenen Boniturwerte werden in Prozent Befall umgerechnet. 0 % bedeutet keinen Befall, 100 % bedeutet, daß die Pflanzen vollständig befallen sind.

In diesem Test zeigen z.B. folgende Verbindungen eine sehr gute Wirkung, die derjenigen der aus dem Stand der Technik bekannten Verbindung( A) deutlich überlegen ist:

Verbindungen gemäß Herstellungsbeispielen 1 und 2.

Patentansprüche

1) Fluorierte 1-Azolyl-butan-Derivate der allgemeinen Formel

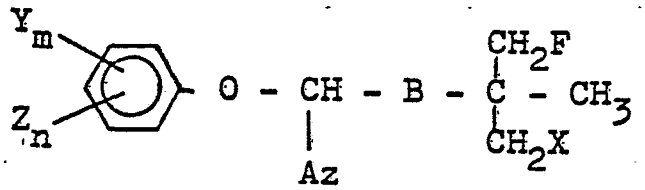

(I)

in welcher

Az für Imidazol-1-yl, 1,2,4-Triazol-1-yl oder 1,2,4-Triazol-4-yl steht,

B für die Ketogruppe oder die CH(OH)-Gruppierung steht,

X für Wasserstoff oder Fluor steht,

Y für Alkoxy, Alkylthio, Halogenalkyl, Halogenalkoxy oder Halogenalkylthio steht,

Z für Halogen oder Alkyl steht,

m für die Zahlen 1, 2 oder 3 steht und

n für die Zahlen 0, 1 oder 2 steht,

sowie deren physiologisch verträglichen Säureadditions-Salze und Metallsalz-Komplexe.

2) Fluorierte 1-Azolyl-butan-Derivate der Formel (I) in Anspruch 1,

wobei

Az für Imidazol-1-yl, 1,2,4-Triazol-1-yl oder 1,2,4-Triazol-4-yl steht,

B für die Ketogruppe oder die CH(OH)-Gruppierung steht,

X für Wasserstoff oder Fluor steht,

Y für Methoxy, Ethoxy, Isopropoxy, tert.-Butyloxy, Methylthio, Trifluormethyl, Chlordifluormethyl, Dichlorfluormethyl, Trichlormethyl, Trifluormethylthio steht,

Z für Fluor, Chlor oder Methyl steht,

m für die Zahlen 1,2 oder 3 steht und

n für die Zahlen 0, 1 oder 2 steht,

sowie deren physiologisch verträglichen Säureadditions-Salze und Metallsalz-Komplexe.

3) Verfahren zur Herstellung von fluorierten 1-Azolylbutan-Derivaten der allgemeinen Formel

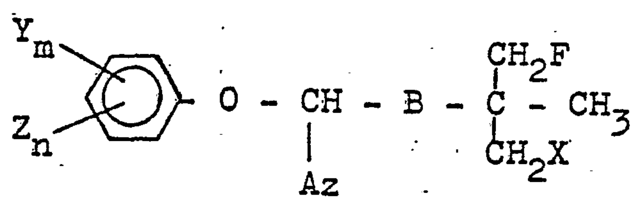

(I)

in welcher

Az für Imidazol-1-yl, 1,2,4-Triazol-1-yl oder 1,2,4-Triazol-4-yl steht,

B für die Ketogruppe oder die CH(OH)-Gruppierung steht,

X für Wasserstoff oder Fluor steht,

Y für Alkoxy, Alkylthio, Halogenalkyl, Halogenalkoxy oder Halogenalkylthio steht,

Z für Halogen oder Alkyl steht,

m für die Zahlen 1, 2 oder 3 steht und

n für die Zahlen 0, 1 oder 2 steht,

sowie deren physiologisch verträglichen Säureadditions-Salzen und Metallsalz-Komplexen, dadurch gekennzeichnet, daß man Halogenetherketone der Formel

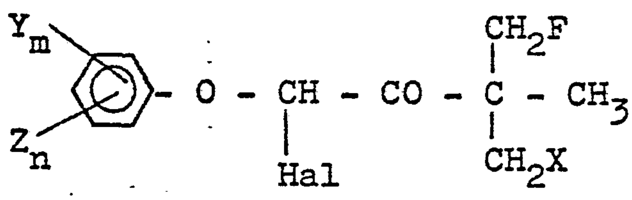

(II)

in welcher

X,Y,Z,n und m die oben angegebene Bedeutung haben und

Hal für Halogen, vorzugsweise Chlor oder Brom, steht,

mit Imidazol oder 1,2,4-Triazol in Gegenwart eines Säurebindemittels und gegebenenfalls in Gegenwart eines Verdünnungsmittels umsetzt und gegebenenfalls die erhaltenen Keto-Derivate nach bekannten Methoden in üblicher Weise reduziert und weiterhin noch an die so erhaltenen Verbindungen der Formel (I) gegebenenfalls anschließend eine Säure oder ein Metallsalz addiert.

4) Fungizide Mittel, gekennzeichnet durch einen Gehalt an mindestens einem fluorierten 1-Azolyl-butan-Derivat der Formel I.

5) Verfahren zur Bekämpfung von Pilzen, dadurch gekennzeichnet, daß man fluorierte 1-Azolyl-butan-Derivate der Formel I auf Pilze oder ihren Lebensraum einwirken läßt.

6) Verwendung von fluorierten 1-Azolyl-butan-Derivaten der Formel I zur Bekämpfung von Pilzen.

7) Verfahren zur Herstellung von fungiziden Mitteln, dadurch gekennzeichnet, daß man fluorierte 1-Azolyl-butan-Derivate der Formel I mit Streckmittel und/ oder oberflächenaktiven Mitteln vermischt.